# EUROPEAN PATENT APPLICATION

(11) **EP 4 674 441 A1**
(43) Date of publication of application: **07.01.2026**
(21) Application number: 23924701.8
(22) Date of filing: 06.03.2023
(51) Int. Cl.: A61K 51/12, A61K 51/06, A61K 51/10, A61K 51/04, A61K 51/02, A61P 35/00

(54) **RARE EARTH NANO FLUORESCENCE-NUCLEAR MEDICINE TUMOR TOMOGRAPHY-THERAPY INTEGRATED PREPARATION, PREPARING METHOD THEREFOR, AND USE METHOD AND USE THEREOF**

(30) Priority: 27.02.2023 CN 202310171218
(71) Applicant: Zhongke Rare Earth Nanotechnology (Hebei) Co., Ltd, Langfang, Hebei 065000 (CN)
(72) Inventor: HONG, Maochun, Fuzhou, Fujian 350002 (CN); CHEN, Hao, Fuzhou, Fujian 350002 (CN); ZHENG, Jingwen, Fuzhou, Fujian 350002 (CN); LIU, Yongsheng, Fuzhou, Fujian 350002 (CN); YE, Lixiang, Fuzhou, Fujian 350002 (CN)
(74) Representative: Gille Hrabal Partnerschaftsgesellschaft mbB Patentanwälte
(86) International application number: PCT/CN2023/079926
(87) International publication number: WO 2024/178735

(57) **Abstract**

Disclosed in the present invention are a rare earth nano fluorescence-nuclear medicine tumor tomography-therapy integrated preparation, a preparing method therefor, and a use method and the use thereof. Said integrated preparation comprises a rare earth inorganic nanocrystal having a microscopic core-shell-shell structure, a rare earth medical nuclide A, and a functional tumor-targeting biomolecule loaded on the surface of the rare earth inorganic nanocrystal, the rare earth medical nuclide A being at least distributed in the rare earth inorganic nanocrystal and/or coupled to the functional tumor-targeting biomolecule. Under the excitation of a 980 nm near-infrared light, said integrated preparation can emit 400-1700 nm rare earth ion fluorescence from the visible region to the near infrared II region, and can further radiate β⁻, β⁺ and γ rays for use in tumor therapy and PET and SPECT, thereby both achieving rare earth nano fluorescence-nuclear medicine multi-modal medical tomography and efficient tumor radioactive therapy for biological living bodies.

## Description

The present application claims priority to the prior application with the patent application No. 202310171218.2, entitled "RARE EARTH NANO FLUORESCENCE-NUCLEAR MEDICINE TUMOR TOMOGRAPHY-THERAPY INTEGRATED PREPARATION, PREPARING METHOD THEREFOR, AND USE METHOD AND USE THEREOF" and filed with the China National Intellectual Property Administration on February 27, 2023, the content of which is incorporated herein by reference in its entirety.

### TECHNICAL FIELD

The present disclosure pertains to the technical field of nanobiomedical imaging-therapy integration, and particularly relates to an integrated imaging-therapy formulation labeled with a radioactive medical isotope based on rare earth nano-fluorescence, single-photon emission computed tomography (SPECT) or positron emission computed tomography, and especially to a rare earth medical nuclide-labeled rare earth nano-fluorescence and nuclear medicine imaging-therapy integrated formulation with a core-shell-shell structure, a preparation method therefor, a use method therefor, and use thereof.

### BACKGROUND

According to the investigation in recent years, malignant tumors have become a major cause of threats to people's health. PET/CT and SPECT/CT diagnoses and treatments taking unique advantages of medical isotopes are indispensable and important means of improving health. Most of the current nuclear medicine imaging agents label targeted biomolecules (such as proteins, polypeptides, monoclonal antibodies, or the like) by medical isotopes. Although such imaging agents have good targeting properties, they usually have a single function and poor stability, and cannot meet the simultaneous diagnosis and treatment requirements of some complex conditions. The integration of diagnosis and treatment by using a rare earth inorganic nano-fluorescent material as a pharmaceutical carrier to load various drug probe molecules is a feasible method for solving the problem.

The rare earth-doped inorganic nano-fluorescent material, as a new generation of fluorescent probe for biological imaging, holds great promise in the field of non-invasive imaging due to its characteristics of low biotoxicity, no background fluorescence, good stability, high imaging resolution, and the like. More importantly, the rare earth inorganic nano-fluorescent material is easy to be doped with rare earth medical radioisotopes (such as ¹⁷⁷Lu, ¹⁶⁹Er, ¹⁵³Sm, ⁸⁶Y, ⁹⁰Y, and the like), easy to modify targeting ligands on the surface, and combined with the luminescence characteristics of the material. Therefore, it can achieve accurate diagnosis and treatment of the lesion simultaneously.

Rare earth medical nuclides have attracted wide attention due to their good medical safety and effectiveness. For example, ¹⁷⁷Lu features a β⁻ ray of 498 keV that can be used in tumor therapy, and can also release a γ ray of 208 keV for SPECT imaging, facilitating the distribution and dose monitoring of radiopharmaceuticals; ⁹⁰Y is pure β⁻ radiation (with an average energy of 0.94 MeV) with an average range of about 1 mm, and has the characteristics of high-energy-density radiation and short range, which can effectively reduce side effects caused by large-area irradiation. However, at present, there are few reports on the use of rare earth medical nuclide-labeled drugs as nanodrugs for integrated diagnosis and treatment of malignant tumors. The conventional labeling method for nuclides uses small molecules to chelate with rare earth cations, resulting in relatively small intermolecular force, insufficient carrier stability, easy off-target effect, and easy damage to normal tissues of organisms.

Therefore, the development of a rare earth nano-fluorescence-nuclear medicine tumor imaging-therapy integrated formulation capable of stably loading medical isotopes and simultaneously performing high-resolution rare earth nano-fluorescence-nuclear medicine multimodal medical imaging and high-efficiency radiotherapy on tumor lesions will be of great practical significance and clinical value in the application of the integration of diagnosis and treatment of diseases.

### SUMMARY

In order to solve the technical problems described above, the present disclosure provides a rare earth nano-fluorescence-nuclear medicine tumor imaging-therapy integrated formulation, a preparation method therefor, and use thereof. The formulation has good biological safety, stability, dispersibility, and uniformity, and can achieve the generation of luminescence from ultraviolet to near-infrared light regions under the excitation of near-infrared light.

In order to achieve the object described above, according to one aspect of the present disclosure, provided is a rare earth nano-fluorescence-nuclear medicine tumor imaging-therapy integrated formulation, comprising a rare earth inorganic nanocrystal with a microscopic core-shell-shell structure, a rare earth medical nuclide A, and a functional tumor-targeting biomolecule loaded on the surface of the rare earth inorganic nanocrystal, wherein the rare earth medical nuclide A is at least distributed inside the microscopic core-shell-shell nanostructure and/or coupled to the functional tumor-targeting biomolecule; the imaging-therapy integrated formulation has a general structural formula of A-MLnF₄@MLnF₄:Em@MLnF₄@LI, wherein
the rare earth medical nuclide A is selected from one or more of ¹⁷⁷Lu, ¹⁶⁹Er, ¹⁵³Sm, ⁹⁰Y, ⁸⁶Y, ⁴⁴Sc, and ⁴⁷Sc;
M is selected from one or more of metal elements Li, Na, K, Rb, Cs, Mg, Ca, Sr, and Ba;
Ln is a stable rare earth element selected from one or more of La, Ce, Pr, Nd, Po, Sm, Eu, Gd, Tb, Dy, Ho, Er, Tm, Yb, Lu, Y, and Sc, preferably one or more of Yb, Er, Tm, Ho, Gd, Y, Ce, and Lu, exemplarily one or more of Gd, Y, Yb, Er, and Ce;
Em is a rare earth luminescent ion selected from one or more of Pr, Nd, Sm, Eu, Tb, Dy, Ho, Er, Tm, and Yb; and
LI is the functional tumor-targeting biomolecule.

The labeling principle of the rare earth medical nuclide is mainly doping and forming a coordination bond between a ligand and the rare earth medical nuclide. The main factors affecting doping include the ionic radius and charge state of the doping ion, and the main factor affecting coordination is the ionic charge state. The doped rare earth medical nuclide and the matrix are both lanthanide elements with similar valence electron structures, and only the size of the ionic radius needs to be considered. The rare earth medical nuclide A of the present disclosure is preferably ¹⁷⁷Lu and ⁹⁰Y.

In one embodiment of the present disclosure, the rare earth inorganic nanocrystal with the microscopic core-shell-shell structure comprises a rare earth ion inner core, a rare earth ion intermediate layer, and a rare earth ion inert protective layer, and the rare earth medical nuclide A is at least distributed on one or more portions of the rare earth ion inner core, the rare earth ion intermediate layer, and the rare earth ion inert protective layer.

The functional tumor-targeting biomolecule is intended to enhance the biocompatibility and tumor-targeting property of an oleic acid-coated rare earth nano-fluorescence-nuclear medicine tumor imaging-therapy integrated formulation precursor. It can be understood by those skilled in the art that the functional tumor-targeting molecule may be selected according to the usage requirements of different tumor types.

In one embodiment of the present disclosure, the functional tumor-targeting biomolecule LI is from a biomolecule with tumor-specific recognition capability coordinated with a water-soluble ligand without tumor-specific recognition capability. In one embodiment of the present disclosure, the water-soluble ligand without tumor-specific recognition capability and the biomolecule with tumor-specific recognition capability are in a mass ratio of (0-100): 1, preferably (0-10):1, for example, 0:1, 1:1, 2:1, 3:1, 4:1, 5:1, 6:1, 7:1, 8:1, 9:1, 10:1, or any range between any of the values described above.

Preferably, the biomolecule with tumor-specific recognition capability is selected from one or more of an organic small molecule, a single- or multi-target small molecule inhibitor, an antibody, and a polypeptide, for example, selected from one or more of a monoclonal antibody, a polyclonal antibody, a polysaccharide, a nucleic acid, folic acid, aspirin, and vitamin C.

In one embodiment of the present disclosure, the water-soluble ligand without tumor-specific recognition capability is selected from an amphiphilic copolymer; the amphiphilic copolymer may be a diblock copolymer and/or a triblock copolymer.

For example, a hydrophilic group in the amphiphilic copolymer is one or more of polyethylene glycol (PEG), monomethoxy poly(ethylene glycol) (mPEG), polyvinylpyrrolidone (PVP), polyoxyethylene (PEO), polyvinyl alcohol (PVA), polyacrylic acid (PAA), chitosan and derivatives thereof, cell membrane-mimetic phosphorylcholine (PC), and water-soluble cyclodextrin derivatives.

In one embodiment of the present disclosure, the polyethylene glycol preferably has a number-average molecular weight of 300-50,000, more preferably 500-10,000, such as 500, 1,000, 2,000, 3,000, 4,000, 5,000, 5,500, 6,000, 6,500, 7,000, 7,500, 8,000, 8,500, 9,000, 9,500, 10,000, 20,000, 25,000, 30,000, 40,000, 50,000, or any range between any two of the values.

In one embodiment of the present disclosure, a hydrophobic group in the amphiphilic copolymer is preferably one or more of polylactic acid, poly-L-lactic acid, poly-D,L-lactic acid, polylactide-glycolide, poly-ε-caprolactone, poly(benzyl aspartate), poly(benzyl glutamate), polystyrene, poly(isopropylacrylamide), polylysine, polyaspartic acid, polyhistidine, phospholipid, phosphatidylethanolamine, distearoyl phosphatidyl ethanolamine, cholesterol, and a hydrophobic cyclodextrin derivative, for example, one or more of polyethylene glycol-polylactic acid (PEG-PLA), polyethylene glycol-poly-D-lactic acid (PEG-PDLA), polyethylene glycol-poly-L-lactic acid (PEG-PLLA), polyethylene glycol-poly-D,L-lactic acid (PEG-PDLLA), polyethylene glycol-polylactide-glycolide (PEG-PLGA), distearoyl phosphatidyl ethanolamine-polyethylene glycol (DSPE-PEG) and derivatives thereof, dipalmitoyl phosphatidyl ethanolamine-polyethylene glycol (DPPE-PEG) and derivatives thereof, dilauroyl phosphatidyl ethanolamine-polyethylene glycol (DLPE-PEG) and derivatives thereof, dimyristoyl phosphatidyl ethanolamine-polyethylene glycol (DMPE-PEG) and derivatives thereof, and dioleoyl phosphatidyl ethanolamine-polyethylene glycol (DOPE-PEG) and derivatives thereof.

In one embodiment of the present disclosure, the imaging-therapy integrated formulation is NaGdF₄@NaYbF₄:Er/Ce/¹⁷⁷Lu@NaYF₄ nanomaterial, NaGdF₄@NaYbF₄:Er/Ce/¹⁶⁹Er@NaYF₄ nanomaterial, NaGdF₄@NaYbF₄:Er/Ce/⁹⁰Y@NaYF₄ nanomaterial, NaGdF₄@NaYbF₄:Er/Ce/¹⁵³Sm@NaYF₄ nanomaterial, NaGdF₄@NaYbF₄:Er/Ce/⁸⁶Y@NaYF₄ nanomaterial, NaGdF₄@NaYbF₄:Er/Ce/⁴⁴Sc@NaYF₄ nanomaterial, NaGdF₄@NaYbF₄:Er/Ce/⁴⁷Sc@NaYF₄ nanomaterial, NaYF₄@NaYbF₄:Er/Ce/¹⁷⁷Lu@NaYF₄ nanomaterial, NaYF₄@NaYbF₄:Er/Ce/¹⁶⁹Er@NaYF₄ nanomaterial, NaYF₄@NaYbF₄:Er/Ce/⁹⁰Y@NaYF₄ nanomaterial, NaYF₄@NaYbF₄:Er/Ce/¹⁵³Sm@NaYF₄ nanomaterial, NaYF₄@NaYbF₄: Er/Ce/⁸⁶Y@NaYF₄ nanomaterial, NaYF₄@NaYbF₄:Er/Ce/⁴⁴Sc@NaYF₄ nanomaterial, NaYF₄@NaYbF₄:Er/Ce/⁴⁷Sc@NaYF₄ nanomaterial, NaGdF₄:¹⁷⁷Lu@NaYbF₄:Er/Ce@NaYF₄ nanomaterial, NaGdF₄:¹⁶⁹Er@NaYbF₄:Er/Ce@NaYF₄ nanomaterial, NaGdF₄:⁹⁰Y@NaYbF₄:Er/Ce@NaYF₄ nanomaterial, NaGdF₄:¹⁵³Sm@NaYbF₄:Er/Ce/@NaYF₄ nanomaterial, NaGdF₄:⁸⁶Y@NaYbF₄:Er/Ce@NaYF₄ nanomaterial, NaGdF₄:⁴⁴Sc@NaYbF₄:Er/Ce@NaYF₄ nanomaterial, NaGdF₄:⁴⁷Sc@NaYbF₄:Er/Ce@NaYF₄ nanomaterial, NaGdF₄@NaYbF₄:Er/Ce@NaYF₄:¹⁷⁷Lu nanomaterial, NaGdF₄@NaYbF₄:Er/Ce@NaYF₄:¹⁶⁹Er nanomaterial, NaGdF₄@NaYbF₄:Er/Ce@NaYF₄:⁹⁰Y nanomaterial, NaGdF₄@NaYbF₄:Er/Ce/@NaYF₄:¹⁵³Sm nanomaterial, NaGdF₄@NaYbF₄:Er/Ce@NaYF₄:⁸⁶Y nanomaterial, NaGdF₄@NaYbF₄:Er/Ce@NaYF₄:⁴⁴Sc nanomaterial, NaGdF₄@NaYbF₄:Er/Ce@NaYF₄:⁴⁷Sc nanomaterial, NaYF₄@NaYF₄:Yb/Er/Ce/¹⁷⁷Lu@NaYF₄ nanomaterial, NaYF₄@NaYF₄:Yb/Er/Ce/¹⁶⁹Er@NaYF₄ nanomaterial, NaYF₄@NaYF₄:Yb/Er/Ce/⁹⁰Y@NaYF₄ nanomaterial, NaYF₄@NaYF₄:Yb/Er/Ce/¹⁵³Sm@NaYF₄ nanomaterial, NaYF₄@NaYF₄:Yb/Er/Ce/⁸⁶Y@NaYF₄ nanomaterial, NaYF₄@NaYF₄:Yb/Er/Ce/⁴⁴Sc@NaYF₄ nanomaterial, NaYF₄@NaYF₄:Yb/Er/Ce/⁴⁷Sc@NaYF₄ nanomaterial, KGdF₄@KYbF₄:Er/Ce/¹⁷⁷Lu@KYF₄ nanomaterial, or LiGdF₄@LiYbF₄:Er/Ce/¹⁷⁷Lu@LⁱYF₄ nanomaterial.

In one embodiment of the present disclosure, the rare earth luminescent ion Em is distributed inside the microscopic core-shell-shell nanostructure of the tumor imaging-therapy integrated formulation.

For example, the rare earth luminescent ion Em is distributed in at least one or more portions of the rare earth ion inner core, the rare earth ion intermediate layer, and the rare earth ion inert protective layer of the nanocrystal with the core-shell-shell structure. In one embodiment of the present disclosure, the functional tumor-targeting biomolecule is located on the surface of the nanocrystal with the core-shell-shell structure. For example, the surface of the nanocrystal is partially or completely coated with the functional tumor-targeting biomolecule.

In one embodiment of the present disclosure, the functional tumor-targeting biomolecule has a thickness of 1-100 nm, preferably 5-50 nm.

In one embodiment of the present disclosure, the rare earth nano-fluorescence-nuclear medicine tumor imaging-therapy integrated formulation has a size of 10-200 nm, preferably 10-100 nm, for example, 10 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, or any range between any of the values described above.

The present disclosure further provides a preparation method for the rare earth nano-fluorescence-nuclear medicine tumor imaging-therapy integrated formulation, and a high-temperature coprecipitation method, a high-temperature thermal decomposition method, a hydrothermal method, or a sol-gel method, preferably a high-temperature thermal decomposition method, may be adopted.

In one embodiment of the present disclosure, the preparation method for the rare earth nano-fluorescence-nuclear medicine tumor imaging-therapy integrated formulation comprises:
S1, obtaining a rare earth ion inner core by a high-temperature thermal decomposition method;
S2, enabling a rare earth ion intermediate layer and a rare earth ion inert protective layer to grow step by step on the surface of the rare earth ion inner core by adopting a layer-by-layer epitaxial growth method; and
S3, labeling a rare earth medical nuclide A to at least a portion of the rare earth ion inner core, the rare earth ion intermediate layer, and the rare earth ion inert protective layer as required, and then loading a functional tumor-targeting biomolecule on the surface of the rare earth ion inert protective layer; or
loading a functional tumor-targeting biomolecule on the surface of the rare earth ion inert protective layer, and then labeling a rare earth medical nuclide A on the functional tumor-targeting biomolecule to obtain the imaging-therapy integrated formulation.

Preferably, the preparation method for the rare earth nano-fluorescence-nuclear medicine tumor imaging-therapy integrated formulation comprises the following steps:
(a) adding a stable rare earth element, an alkali metal source, a long-alkyl-chain organic acid, and a long-alkyl-chain olefin into a reaction vessel, mixing until dissolved, then heating to a certain temperature, and naturally cooling to room temperature after a period of reaction to obtain a rare earth ion inner core;
(b) adding the rare earth ion inner core, the stable rare earth element, the alkali metal source, the long-alkyl-chain organic acid, and the long-alkyl-chain olefin into a reaction vessel, mixing until dissolved, then heating to a certain temperature, reacting for a period of time, and naturally cooling to room temperature to obtain a nanocrystal with a core-shell structure;
(c) adding the nanocrystal with the core-shell structure, the stable rare earth element, the alkali metal source, the long-alkyl-chain organic acid, and the long-alkyl-chain olefin into a reaction vessel, mixing until dissolved, then heating to a certain temperature for reaction, and naturally cooling to room temperature to obtain a rare earth nano-fluorescence-nuclear medicine tumor imaging-therapy integrated formulation precursor with a core-shell-shell structure, wherein at this time, the surface of the nanoparticle is coated with a layer of oleic acid ligands, which is beneficial to enhancing the stability of the nanoparticle; and
(d) modifying the surface of the imaging-therapy integrated formulation precursor with the functional tumor-targeting biomolecule,

wherein the rare earth medical nuclide A is loaded in at least one process of step (a), step (b), step (c), and step (d), and
wherein a rare earth luminescent ion Em is added in at least one process of step (a), step (b), and step (c).

Preferably, the rare earth luminescent ion Em is added in step (b).

Preferably, the temperature is raised to 280-340 °C, and the reaction is performed for 0.5-2 h in step (a), step (b), and step (c).

Preferably, the step of modifying with the functional tumor-targeting biomolecule in step (d) comprises:
dispersing the rare earth nano-fluorescence-nuclear medicine tumor imaging-therapy integrated formulation precursor in an organic solvent, adding distearoyl phosphatidyl ethanolamine-polyethylene glycol (DSPE-PEG) and a polypeptide derivative thereof, stirring for a period of time to convert the nanocrystal from oil-soluble to water-soluble, completely volatilizing the solvent naturally, and then adding a proper amount of water for ultrasonic mixing to obtain the rare earth nano-fluorescence-nuclear medicine tumor imaging-therapy integrated formulation.

In one embodiment of the present disclosure, the stable rare earth element is selected from one or more of an acetate, a trifluoroacetate, a chloride salt, and a nitrate of a rare earth ion stable isotope Ln, preferably a trifluoroacetate and a rare earth chloride salt. Preferably, the stable rare earth element is selected from trifluoroacetates and/or chloride salts of ytterbium, erbium, gadolinium, thulium, holmium, samarium, neodymium, yttrium, and lutetium, exemplarily ytterbium trifluoroacetate, yttrium trifluoroacetate, erbium trifluoroacetate, cerium trifluoroacetate, and gadolinium trifluoroacetate.

In one embodiment of the present disclosure, the alkali metal source provides an element M for the nanocrystal with the core-shell-shell structure.

In one embodiment of the present disclosure, the alkali metal source is selected from one or more of salts, hydroxides, and bifluorides containing the M element; M is selected from one or more of metal elements Li, Na, K, Rb, Cs, Mg, Ca, Sr, and Ba.

Preferably, the alkali metal source is an oleate, an acetate, or a fluoride containing the M element, such as a trifluoroacetate, exemplarily sodium trifluoroacetate or sodium bifluoride.

In one embodiment of the present disclosure, the long-alkyl-chain organic acid is selected from one or more of octanoic acid, lauric acid, and oleic acid, exemplarily oleic acid.

In one embodiment of the present disclosure, the long-alkyl-chain olefin is selected from one or more of the following: 1-decene, 1-dodecene, and 1-octadecene, exemplarily 1-octadecene.

In one embodiment of the present disclosure, the rare earth medical nuclide A is selected from an oxalate of a rare earth radioisotope and/or a chloride salt of a rare earth radioisotope, exemplarily ¹⁷⁷LuCl₃ and/or ⁹⁰YCl₃.

In one embodiment of the present disclosure, the stable rare earth element, the long-alkyl-chain organic acid, and the long-alkyl-chain olefin are in a molar volume ratio of (0.01-0.4) mmol:(1-20) mL:(1-20) mL, preferably (0.01-0.2) mmol:(2-10) mL:(2-10) mL.

In one embodiment of the present disclosure, the rare earth medical nuclide A and the stable rare earth element are in a radioactivity-to-molar ratio of **(0.01-100) mCi:(0.01-**0.4) mmol, preferably (1-50) mCi:(0.01-0.1) mmol.

In one embodiment of the present disclosure, the organic solvent is one of cyclohexane, dichloromethane, trichloromethane, tetrahydrofuran, and N,N-dimethylformamide, or a mixed solvent thereof.

In one embodiment of the present disclosure, the nanocrystal, distearoyl phosphatidyl ethanolamine-polyethylene glycol and the polypeptide derivative of distearoyl phosphatidyl ethanolamine-polyethylene glycol, cyclohexane, and trichloromethane are in a molar volume ratio of (0.01-0.2) mmol:(0.2-0.8) mmol:(1-10) mL:(2.5-10) mL, preferably (0.05-0.1) mmol:(0.3-0.5) mmol:(1-3) mL:(8-10) mL.

In one embodiment of the present disclosure, step (d) is followed by steps of precipitating and washing the reaction solution. Preferably, the precipitation is performed by high-speed centrifugation.

In one embodiment of the present disclosure, step (d) is followed by a step of redispersing the washed product, e.g., in normal saline. Preferably, step (d) is followed by a step of performing sterile filtration on the dispersed solution.

The present disclosure further provides use of the rare earth nano-fluorescence-nuclear medicine imaging-therapy integrated formulation described above as a rare earth fluorescent medical imaging agent for solid tumor lesions or small and micro metastatic tumor lesions (< 1 mm).

The present disclosure further provides use of the rare earth nano-fluorescence-nuclear medicine imaging-therapy integrated formulation described above as a nuclear medicine SPECT tumor medical imaging agent.

The present disclosure further provides use of the rare earth nano-fluorescence-nuclear medicine imaging-therapy integrated formulation described above as an efficient radioactive therapeutic (radiotherapeutic) agent for tumors.

The present disclosure further provides use of the rare earth nano-fluorescence-nuclear medicine tumor imaging-therapy integrated formulation described above as the integration of tumor diagnosis and treatment under the combined guidance of rare earth nano-fluorescence-nuclear medicine multimodal medical imaging.

### Beneficial Effects of Present Disclosure:

In the present disclosure, the lanthanide rare earth is used as a matrix material, a rare earth radioactive medical nuclide with radioactivity is incorporated into a crystal lattice thereof by adopting an improved high-temperature thermal decomposition method to obtain a rare earth nano-fluorescence-nuclear medicine tumor imaging-therapy integrated formulation precursor with radioactivity, and then the precursor is functionally modified to prepare a rare earth nano-fluorescence-nuclear medicine tumor imaging-therapy integrated formulation, which has the following advantages:
1) The synthesis method is easy to control, and has good repeatability and high universality, and the prepared radioactive medical nuclide-labeled rare earth nano-fluorescence-nuclear medicine tumor imaging-therapy integrated formulation has good dispersibility and uniformity, and can achieve the generation of luminescence from ultraviolet to near-infrared light regions under the excitation of near-infrared light.
2) The lanthanide rare earth-based nano-fluorescent material is easy to be doped with rare earth medical isotopes; for example, ¹⁷⁷Lu is a radioisotope prepared by subjecting ¹⁷⁶Yb to reactor irradiation and then to β⁻ decay, the two elements have similar physical properties such as ionic radius, charge state, and the like, and are easy to be doped in a crystal lattice, and the medical isotope doped in the crystal lattice can be stably present in the crystal material, resulting in better stability, less off-target effect, and higher biological safety.
3) The surface of the rare earth nano-fluorescence-nuclear medicine tumor imaging-therapy integrated formulation precursor is easy to modify and can be selectively covered with various functional tumor-targeting biomolecules, thereby meeting different demand scenarios in actual situations.
4) The rare earth nano-fluorescence-nuclear medicine diagnostic and therapeutic agent prepared by the present disclosure exhibits excellent radiochemical stability and good biological safety, can be safely used in organisms, can be used as a brand-new inorganic nano- fluorescence/nuclear medicine imaging material, and has a wide application prospect in the fields of drug transportation, biological imaging, tumor treatment, and the like. By loading rare earth medical nuclides, accurate diagnosis and treatment of lesions under high-resolution rare earth fluorescence-nuclear medicine multimodal medical image navigation are simultaneously achieved, which will be of great practical significance and clinical value in the diagnosis and treatment application of diseases.
5) Under the excitation of 980-nm near-infrared light, the tumor imaging-therapy integrated formulation can generate rare earth ion fluorescence emission from the visible region to the near-infrared II region at 400-1700 nm, and can further radiate β⁻, β⁺, and γ rays for tumor treatment and nuclear medicine imaging, thereby both achieving rare earth nano-fluorescence-nuclear medicine multimodal medical imaging and efficient tumor radioactive therapy (radiotherapy) for living organisms.

### BRIEF DESCRIPTION OF THE DRAWINGS

FIG. 1 shows X-ray powder diffraction patterns of β-NaGdF₄, NaGdF₄@NaYbF₄:Er/Ce/¹⁷⁷Lu, and NaGdF₄@NaYbF₄:Er/Ce/¹⁷⁷Lu@NaYF₄ nanoparticles prepared in Example 1 according to the present disclosure.
FIG. 2 shows a transmission electron micrograph and a particle size distribution histogram of NaGdF₄@NaYbF₄:Er/Ce/¹⁷⁷Lu@NaYF₄ nanoparticles prepared in Example 1 according to the present disclosure.
FIG. 3 shows a transmission electron micrograph of NaGdF₄@NaYbF₄:Er/Ce/¹⁷⁷Lu@NaYF₄ nanoparticles modified with a tumor-targeting biomolecule in Example 1 according to the present disclosure.
FIG. 4 shows a hydrodynamic particle size map of NaGdF₄@NaYbF₄:Er/Ce/¹⁷⁷Lu@NaYF₄@LI nanoparticles modified with a tumor-targeting biomolecule in Example 1 according to the present disclosure.
FIG. 5 shows a Zeta potential diagram of NaGdF₄@NaYbF₄:Er/Ce/¹⁷⁷Lu@NaYF₄@LI nanoparticles modified with a tumor-targeting biomolecule in Example 1 according to the present disclosure.
FIG. 6 shows an X-ray powder diffraction pattern of NaGdF₄@NaYbF₄:Er/Ce@NaYF₄:⁹⁰Y nanoparticles prepared in Example 2 according to the present disclosure.
FIG. 7 shows a transmission electron micrograph and a particle size distribution histogram of NaGdF₄@NaYbF₄:Er/Ce@NaYF₄:⁹⁰Y nanoparticles prepared in Example 2 according to the present disclosure.
FIG. 8 shows a transmission electron micrograph of NaGdF₄@NaYbF₄:Er/ Ce@NaYF₄:⁹⁰Y nanoparticles modified with a tumor-targeting biomolecule in Example 2 according to the present disclosure.
FIG. 9 is a schematic diagram showing the radiochemical stability of the NaGdF₄@NaYbF₄:Er/Ce/¹⁷⁷Lu@NaYF₄@LI tumor imaging-therapy integrated formulation in Example 1 dispersed in normal saline and fetal bovine serum in Experimental Example 1 according to the present disclosure.
FIG. 10 is a schematic SPECT image showing the distribution of the NaGdF₄@NaYbF₄:Er/Ce/¹⁷⁷Lu@NaYF₄@LI tumor imaging-therapy integrated formulation prepared in Example 1 in nude mice 1 h, 24 h, 48 h, and 96 h after administration through the tail vein in Experimental Example 2 according to the present disclosure.
FIG. 11 is a schematic SPECT image showing the intratumoral retention of the NaGdF₄@NaYbF₄:Er/Ce/¹⁷⁷Lu@NaYF₄@LI tumor imaging-therapy integrated formulation in Example 1 in nude mice 1 h, 24 h, 48 h, and 96 h after intratumoral administration in Experimental Example 2 according to the present disclosure.
FIG. 12 is a schematic diagram showing the fluorescence imaging effect of the NaGdF₄@NaYbF₄:Er/Ce/¹⁷⁷Lu@NaYF₄@LI tumor imaging-therapy integrated formulation in Example 1 in nude mice 1 h, 24 h, 48 h, and 96 h after administration through the tail vein in Experimental Example 3 according to the present disclosure.
FIG. 13 is a schematic diagram showing the surgical navigation effect of the NaGdF₄@NaYbF₄:Er/Ce/¹⁷⁷Lu@ NaYF₄@LI tumor imaging-therapy integrated formulation in Example 1 in nude mice 48 h after intraperitoneal administration in Experimental Example 4 according to the present disclosure.
FIG. 14 shows the tumor-targeting fluorescence imaging effect of the NaGdF₄@NaYbF₄:Er/Ce/¹⁷⁷Lu@ NaYF₄@LI tumor imaging-therapy integrated formulation in Example 1 on tumor cells *in vitro* in Experimental Example 5 according to the present disclosure.
FIG. 15 shows the binding of the NaGdF₄@NaYbF₄:Er/Ce/¹⁷⁷Lu@ NaYF₄@LI tumor imaging-therapy integrated formulation in Example 1 to U87MG cells in Experimental Example 5 according to the present disclosure.
FIG. 16 shows the killing effects of the NaGdF₄@NaYbF₄:Er/Ce@NaYF₄:⁹⁰Y@LI tumor imaging-therapy integrated formulation in Example 2, NaGdF₄@NaYbF₄:Er/Ce@NaYF₄@ LI in Comparative Example 1, and ⁹⁰YCl₃ on *in vitro* U87MG tumor cells in Experimental Example 5 according to the present disclosure.
FIG. 17 shows the killing effects of the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce@ NaYF₄@LI in Comparative Example 1, and ¹⁷⁷LuCl₃ on *in vitro* U87MG tumor cells in Experimental Example 5 according to the present disclosure.
FIG. 18 shows the volume change curves of tumors in each group during the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce/ @NaYF₄@LI in Comparative Example 1, ¹⁷⁷LuCl₃, and normal saline in Experimental Example 5 according to the present disclosure.
FIG. 19 shows photographs of representative nude mice in each group on day 7 and day 15 during the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce@NaYF₄@LI in Comparative Example 1, ¹⁷⁷LuCl₃, and normal saline in Experimental Example 5 according to the present disclosure.
FIG. 20 shows HE staining images of representative tumor tissues of each group after the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce @NaYF₄@LI in Comparative Example 1, ¹⁷⁷LuCl₃, and normal saline in Experimental Example 5 according to the present disclosure.
FIG. 21 shows TUNEL results of representative tumor tissues of each group after the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce @NaYF₄@LI in Comparative Example 1, ¹⁷⁷LuCl₃, and normal saline in Experimental Example 5 according to the present disclosure.
FIG. 22 shows the body weight change curves of nude mice in each group during the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce@NaYF₄@LI in Comparative Example 1, ¹⁷⁷LuCl₃, and normal saline in Experimental Example 6 according to the present disclosure.
FIG. 23 shows HE staining images of representative heart, liver, spleen, lung, and kidney tissues of each group after the treatment of nude mice with the NaGdF₄@NaYbF₄:Er/Ce/¹⁷⁷Lu@ NaYF₄@LI tumor imaging-therapy integrated formulation in Example 1, NaGdF₄@NaYbF₄:Er/Ce @NaYF₄@LI in Comparative Example 1, ¹⁷⁷LuCl₃, and normal saline in Experimental Example 6 according to the present disclosure.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the raw materials and reagents used in the following examples are all commercially available products or can be prepared by using known methods. The instruments involved in the following examples are as follows:
An X-ray powder diffractometer (instrument model: MiniFlex2, manufacturer: Rigaku), a transmission electron microscope (instrument model: TECNAI G2 F20, manufacturer: FEI), a laser particle analyzer (instrument model: Zetasizer Ultra, manufacturer: Malvern), an intelligent calibrator (instrument model: China Nuclear FH463B, manufacturer: Guangzhou Yitongxing Instrument Co., Ltd.), a small animal *in vivo* fluorescence imaging system (instrument model: Ninox 640 SU, manufacturer: Raptor Photonics), a confocal microscope (instrument model: Al+, manufacturer: Nikon), and a single photon emission/X-ray transmission computed tomographic scanner for living small animals (instrument model: IRIS SPECT/ CT, manufacturer: Inviscan).

### Example 1: Preparation of NaGdF₄@NaYbF₄:Er/Ce/¹⁷⁷Lu@NaYF₄ Nanomaterial

### (1) Preparation of NaGdF₄@NaYbF₄:Er/Ce/¹⁷⁷Lu@NaYF₄ nanoparticles based on thermal decomposition method

(a) 0.1360 g of sodium trifluoroacetate and 0.4960 g of gadolinium trifluoroacetate were weighed out and added to a three-necked flask at room temperature, and 3 mL of oleic acid, 3 mL of oleylamine, and 6.4 mL of 1-octadecene were added as a mixed solvent; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain an α-phase NaGdF₄ nanocrystal.
(b) The α-phase NaGdF₄ was added to a mixed solvent of 6.4 mL of oleic acid and 6.4 mL of 1-octadecene, and 0.0680 g of sodium trifluoroacetate and 0.2480 g of gadolinium trifluoroacetate were added; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain a β-phase NaGdF₄ nanocrystal, with a particle size of about 8 nm.
(c) The β-phase NaGdF₄ was added to a mixed solvent of 6.4 mL of oleic acid and 6.4 mL of 1-octadecene, and 0.01360 g of sodium trifluoroacetate, 0.0481 g of ytterbium trifluoroacetate, 0.0010 g of erbium trifluoroacetate, 0.0013 g of cerium trifluoroacetate, and 3 mCi ¹⁷⁷LuCl₃ were added; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain a NaGdF₄@NaYbF₄:Er/Ce/¹⁷⁷Lu nanocrystal, with a particle size of about 12 nm.
(d) NaGdF₄@NaYbF₄:Er/Ce/¹⁷⁷Lu was added to a mixed solvent of 6.4 mL of oleic acid and 6.4 mL of 1-octadecene at room temperature, and 0.0136 g of sodium trifluoroacetate and 0.0428 g of yttrium trifluoroacetate were added; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain a NaGdF₄@NaYbF₄:Er/Ce/¹⁷⁷Lu@NaYF₄ nanocrystal, which was dispersed in 10 mL of a cyclohexane solution for later use.

### (2) Coating NaGdF₄@NaYbF₄:Er/Ce/¹⁷⁷Lu@NaYF₄ nanocrystal with functional tumor-targeting biomolecule:

1 mL of the prepared NaGdF₄@NaYbF₄:Er/Ce/¹⁷⁷Lu@NaYF₄ nanocrystal was dispersed in a mixed solution of 2 mL of cyclohexane and 10 mL of trichloromethane at room temperature, and distearoyl phosphatidyl ethanolamine-polyethylene glycol (DSPE-PEG) and a polypeptide derivative thereof with a mass 10 times that of the nanocrystal were rapidly added under stirring until the solvent was completely and naturally volatilized. A proper amount of purified water was added, and the resulting solid was redispersed. The reaction solution was precipitated by high-speed centrifugation and washed to obtain a precipitation product, namely a rare earth nano-fluorescence-nuclear medicine tumor imaging-therapy integrated formulation with a core-shell-shell structure, which was dispersed and stored in normal saline.

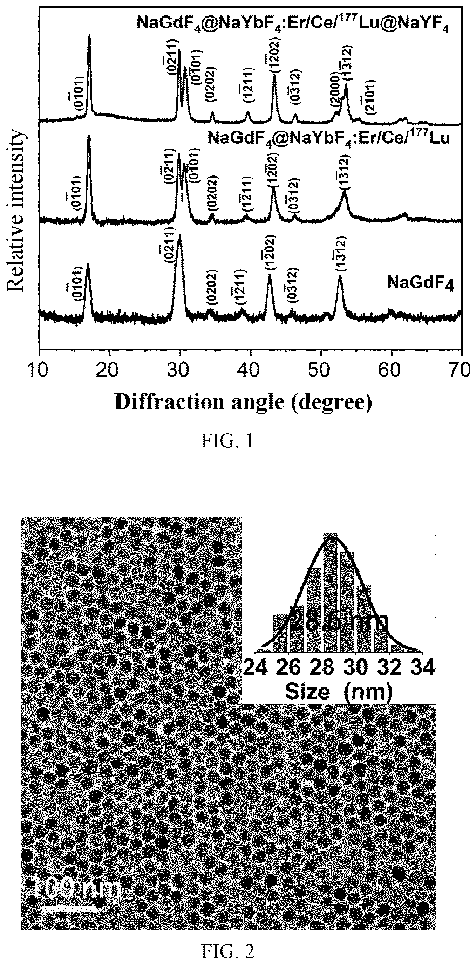

FIG. 1 shows X-ray powder diffraction patterns of β-NaGdF₄, NaGdF₄@NaYbF₄:Er/Ce/¹⁷⁷Lu, and NaGdF₄@NaYbF₄:Er/Ce/¹⁷⁷Lu@NaYF₄ nanocrystals prepared in Example 1 according to the present disclosure.

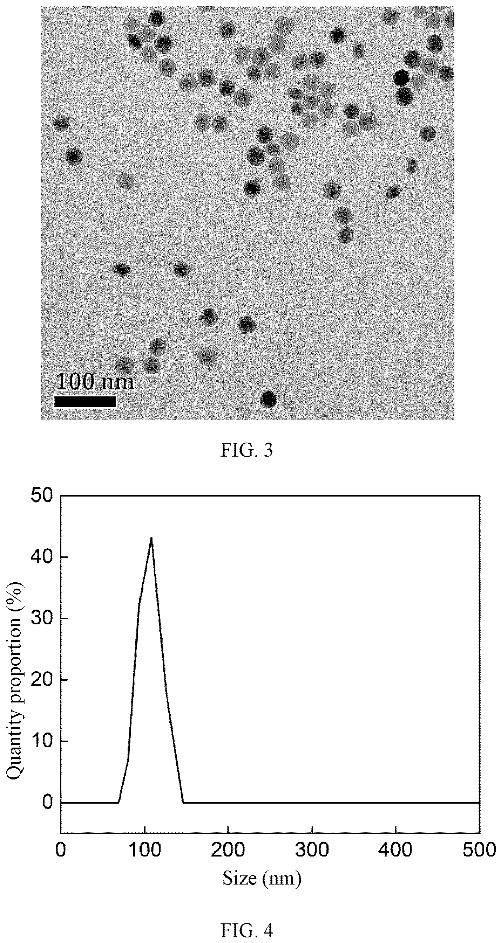

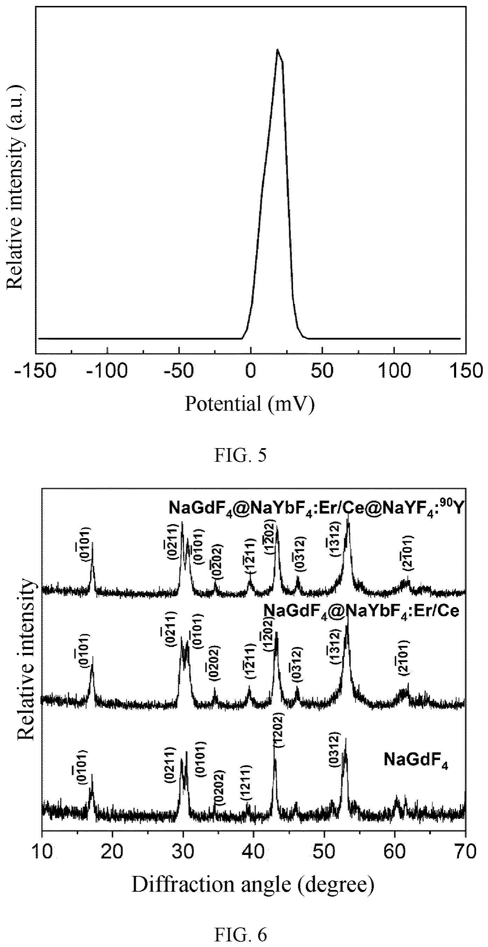

FIG. 2 shows a transmission electron micrograph and a particle size distribution pattern of the NaGdF₄@NaYbF₄:Er/Ce/¹⁷⁷Lu@NaYF₄ nanoparticles prepared in Example 1. The prepared ¹⁷⁷Lu-labeled rare earth nano-fluorescence-nuclear medicine tumor imaging-therapy integrated formulation has good dispersibility, the size of the nanoparticles is about 28.6 nm (see FIG. 3), the hydrodynamic particle size is 109 nm (see FIG. 4), and the Zeta potential is 18.5 mV (see FIG. 5).

### Example 2: Preparation of NaGdF₄@NaYbF₄:Er/Ce@NaYF₄:⁹⁰Y@LI Nanomaterial

### (1) Preparation of NaGdF₄@NaYbF₄:Er/Ce@NaYF₄:⁹⁰Y nanoparticles based on thermal decomposition method

The synthesis procedures of (a) and (b) were the same as those of Example 1.

(c) The β-phase NaGdF₄ was added to a mixed solvent of 6.4 mL of oleic acid and 6.4 mL of 1-octadecene, and 0.01360 g of sodium trifluoroacetate, 0.0481 g of ytterbium trifluoroacetate, 0.0010 g of erbium trifluoroacetate, and 0.0013 g of cerium trifluoroacetate were added; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain a NaGdF₄@NaYbF₄:Er/Ce nanocrystal.

(d) NaGdF₄@NaYbF₄:Er/Ce was added to a mixed solvent of 6.4 mL of oleic acid and 6.4 mL of 1-octadecene at room temperature, and 0.0136 g of sodium trifluoroacetate, 0.0428 g of yttrium trifluoroacetate, and 3 mCi ⁹⁰YCl₃ were added; the mixture was heated under vacuum until the trifluoroacetates described above were dissolved, and then the mixture was heated to 300 °C under an inert atmosphere; after 0.5 h of reaction, the mixture was naturally cooled to room temperature, precipitated, and washed to obtain a NaGdF₄@NaYbF₄:Er/Ce@NaYF₄:⁹⁰Y nanocrystal, which was dispersed in 10 mL of a cyclohexane solution for later use.

### (2) Coating NaGdF₄@NaYbF₄:Er/Ce@NaYF₄:⁹⁰Y nanocrystal with functional tumor-targeting biomolecule:

The synthesis procedure was the same as that of Example 1.

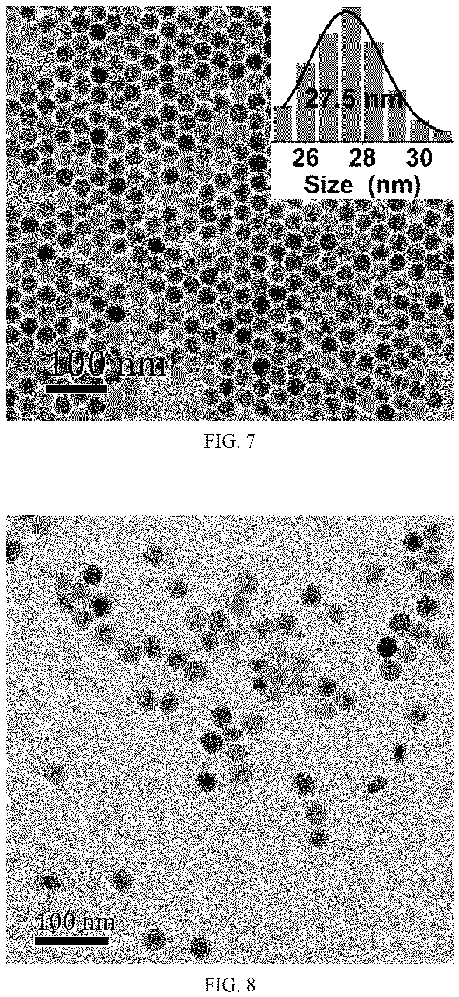

The prepared ⁹⁰Y-labeled rare earth nano-fluorescence-nuclear medicine tumor imaging-therapy integrated formulation precursor has good crystallinity (see FIG. 6) and dispersibility (see FIG. 7), is still in a monodisperse state after targeting molecule modification, and has good water solubility and stability (see FIG. 8).

### Comparative Example 1: Preparation of NaGdF₄@NaYbF₄:Er/Ce@NaYF₄@LI Nanomaterial

The synthesis procedure of Comparative Example 1 was the same as that of Example 1 except that ¹⁷⁷LuCl₃ was not added.

### Experimental Example 1

Test for radiostability of NaGdF₄@NaYbF₄:Er/Ce/¹⁷⁷Lu@NaYF₄@LI rare earth nano-fluorescence-nuclear medicine tumor imaging-therapy integrated formulation prepared in Example 1:
1.5 MBq of NaGdF₄@NaYbF₄:Er/Ce/¹⁷⁷Lu@NaYF₄@LI was mixed thoroughly with an equal volume of normal saline and fetal bovine serum. The mixture was incubated at 37 °C, and centrifuged at 0.5 h, 1 h, 2 h, 4 h, 8 h, 12 h, 24 h, and 48 h using an ultrafiltration tube to collect the subnatant. The radiostability of the material was analyzed using an intelligent calibrator.

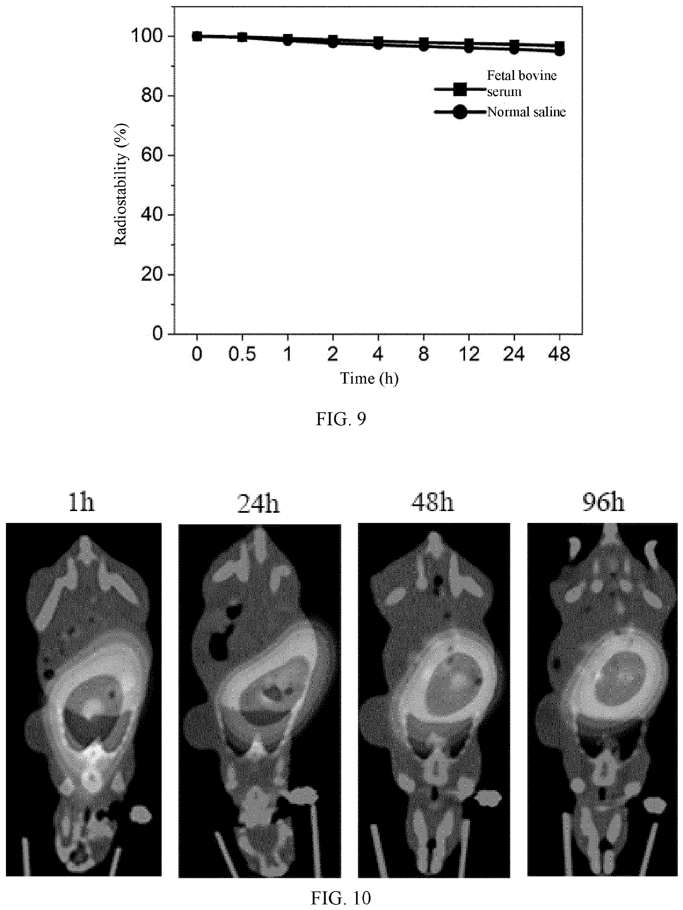

As shown in FIG. 9, it can be seen that the rare earth nano-fluorescence-nuclear medicine tumor imaging-therapy integrated formulation prepared in Example 1 according to the present disclosure had good stability in both normal saline and fetal bovine serum, and the activity after incubation at 37 °C for 48 h was 94.96% and 96.80% of the initial activity, respectively.

### Experimental Example 2

SPECT imaging effect of NaGdF₄@NaYbF₄:Er/Ce/¹⁷⁷Lu@NaYF₄@LI rare earth nano-fluorescence-nuclear medicine tumor imaging-therapy integrated formulation prepared in Example 1:

### (1) Tumor-bearing nude mouse model

The nude mouse experiment was approved by the Animal Protection Committee of Sichuan University. Tumor cells (about 5 × 10⁶) resuspended in 100 µL of PBS were injected subcutaneously or intraperitoneally into BALB/C nude mice (male, aged 4 weeks, Beijing Vital River Laboratory Animal Technology Co., Ltd., China). The tumor models of the present disclosure were all established according to this method, and the tests were performed after the tumor volume reached about 100 mm³.

### (2) Observation of distribution and metabolism in tumor-bearing nude mice by tail vein injection

The rare earth nano-fluorescence-nuclear medicine tumor imaging-therapy integrated formulation prepared in Example 1 with a dose of 150 mg/kg and a radioactive dose of 5 mCi/kg was injected intravenously into the tail vein of nude mice bearing U87MG subcutaneous tumors with a body weight of about 20 g, and SPECT imaging was performed at time points of 1 h, 24 h, 48 h, and 96 h to observe the *in vivo* distribution and metabolism of the formulation.

As can be seen from FIG. 10, the rare earth nano-fluorescence-nuclear medicine tumor imaging-therapy integrated formulation injected in the first hour was mainly distributed above the abdomen of the nude mice, and should be accumulated at the liver site, but could not be accurately positioned, which was a limitation of SPECT image; as time passed, there were also certain image signals in the lower abdomen; it was worth noting that in the 24-h image, there were significant signals in the stomach; there were also signals in the intestinal tract at 48 h, indicating that part of the tumor diagnostic and therapeutic agent was metabolized through the digestive tract after being injected into the tail vein; the signals after 96 h began to gradually weaken, indicating that a considerable part of the tumor diagnostic and therapeutic agent had been metabolized out of the nude mice.

### (3) Observation of retention at tumor site in tumor-bearing nude mice by intratumoral injection

The rare earth nano-fluorescence-nuclear medicine tumor imaging-therapy integrated formulation prepared in Example 1 with a dose of 150 mg/kg and a radioactive dose of 5 mCi/kg was injected intratumorally into nude mice bearing U87MG subcutaneous tumors with a body weight of about 20 g, and SPECT imaging was performed at time points of 1 h, 24 h, 48 h, and 96 h to observe the retention of the formulation at the tumor site.

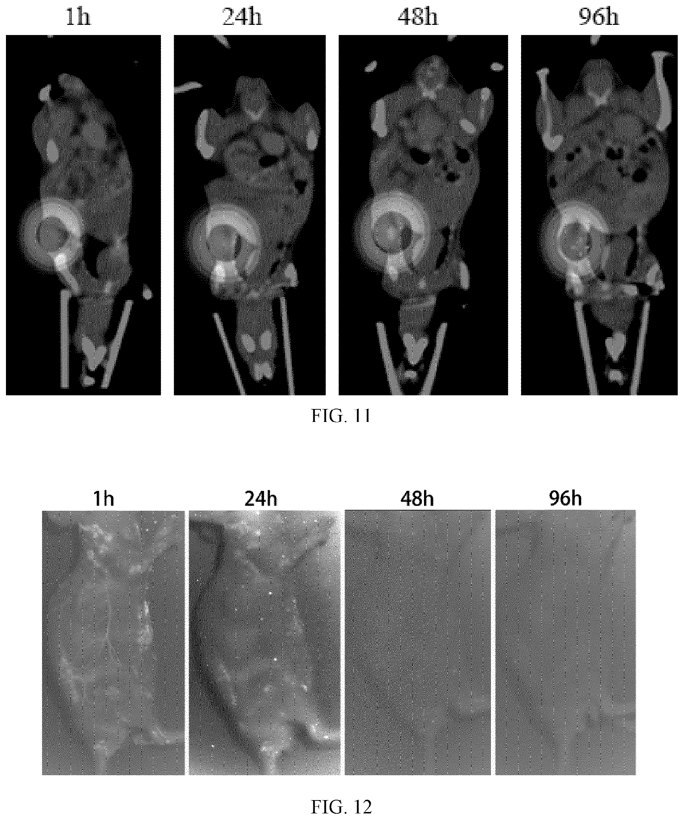

After intratumoral injection, the prepared rare earth nano-fluorescence-nuclear medicine tumor imaging-therapy integrated formulation could maintain high-concentration aggregation at the tumor site for a long time. The imaging results at 1 h, 24 h, 48 h, and 96 h (FIG. **11****)** show that the tumor tissue was the main drug distribution site, and other normal tissues and organs exhibited almost no radioactive distribution from 12 h to 96 h after injection, indicating that the tumor diagnostic and therapeutic agent has good tumor retention ability by intratumoral injection, and also indicating that the tumor diagnostic and therapeutic agent has good stability *in vivo.*

### Experimental Example 3

### Fluorescence imaging effect of NaGdF₄@NaYbF₄:Er/Ce/¹⁷⁷Lu@NaYF₄@LI rare earth nano-fluorescence-nuclear medicine tumor imaging-therapy integrated formulation:

The fluorescence-SPECT tumor imaging-therapy integrated formulation prepared in Example 1 with a dose of 150 mg/kg was injected intravenously into the tail vein of nude mice bearing U87MG subcutaneous tumors, and fluorescence imaging was performed at time points of 1 h, 24 h, 48 h, and 96 h to observe the *in vivo* fluorescence imaging effect, as well as distribution and metabolism of the formulation.

Under a small animal *in vivo* fluorescence imaging system, imaging was performed by the excitation of a 980-nm laser, and the results are shown in FIG. 12. Within the first hour of injection, blood vessels in the lateral abdomen of nude mice could be clearly observed, and even capillaries in the thighs could be observed. The resolution is between tens of microns, which is the advantage of fluorescence imaging. However, it should also be recognized that although the resolution is high enough, the penetration depth is limited, and the blood vessel distribution of organs *in vivo* cannot be observed. In terms of the distribution, the formulation was mainly distributed throughout the blood of nude mice at the beginning, and a small part of the formulation was enriched in brown fat. 24 h after injection, substantially no fluorescence signal was observed in the blood, and the formulation was mainly distributed in organs such as the liver, spleen, stomach, intestine, bladder, and the like, and had begun to be metabolized out of the body, which was substantially consistent with the experimental results observed by SPECT. By 96 h, substantially no signal was seen in the lateral decubitus position, indicating that most of the rare earth nano-fluorescence-nuclear medicine tumor imaging-therapy integrated formulation had been metabolized out of the body.

### Experimental Example 4

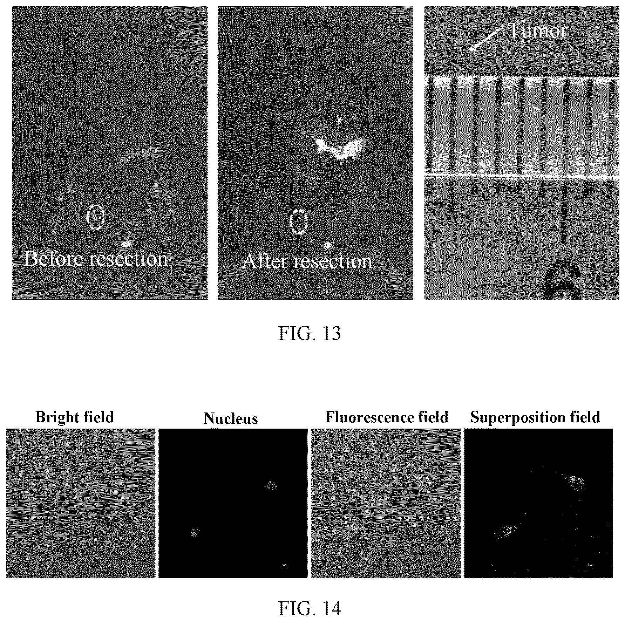

Surgical navigation effect of NaGdF₄@NaYbF₄:Er/Ce/¹⁷⁷Lu@NaYF₄@LI rare earth nano-fluorescence-nuclear medicine tumor imaging-therapy integrated formulation: The rare earth nano-fluorescence-nuclear medicine tumor imaging-therapy integrated formulation prepared in Example 1 with a dose of 150 mg/kg and a radioactive dose of 5 mCi/kg was injected intraperitoneally into nude mice bearing MGC803 metastatic tumors. After 48 h, the micrometastatic tumor was resected under fluorescence imaging navigation. The surgical effect is shown in FIG. 13. The position of the tumor was accurately determined according to the fluorescence signal before the resection, and then the tumor was accurately resected by microsurgery. After the resection, the fluorescence signal at the tumor site disappeared, indicating that the tumor was resected very cleanly. The final size of the resected tumor was 1 mm or less.

### Experimental Example 5

Tumor-targeting property and anti-tumor effect of rare earth nano-fluorescence-nuclear medicine tumor imaging-therapy integrated formulation

### (1) Test for tumor-targeting property of nanomaterial by NaGdF₄@NaYbF₄:Er/Ce/¹⁷⁷Lu@NaYF₄@LI cell fluorescence imaging

U87MG cells were seeded in a culture dish at a density of 1 × 10⁴ cells/well. After the cells completely adhered to the wall, 20 µL of NaGdF₄@NaYbF₄:Er/Ce/¹⁷⁷Lu@NaYF₄ at a concentration of 10 mg/mL was added. After incubation at 37 °C for a period of time, the old medium was discarded, and the cells were washed twice with PBS, fixed with 4% paraformaldehyde for 5 min, washed twice with PBS, stained with DAPI for 2 min, washed three times with PBS, and observed for cell fluorescence under a confocal microscope.

As can be seen from FIG. 14, the rare earth nano-fluorescence-nuclear medicine tumor imaging-therapy integrated formulation had good targeting ability on U87MG tumor cells, and the tumor imaging-therapy integrated formulation could enter the cytoplasm in a short time.

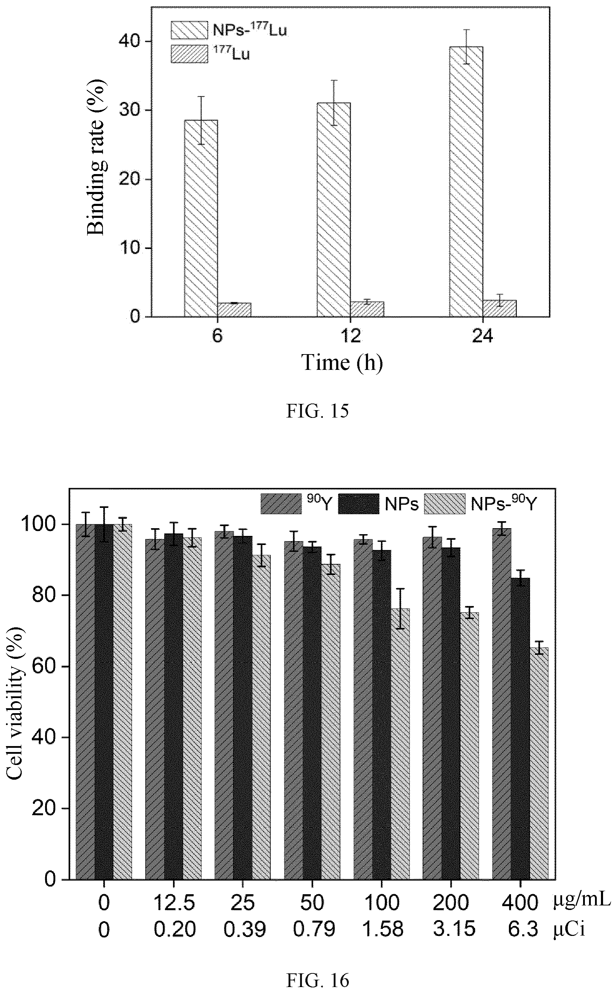

*(2) In vitro* cell binding assay of NaGdF₄@NaYbF₄:Er/Ce/¹⁷⁷Lu@NaYF₄@LI NaGdF₄@NaYbF₄:Er/Ce/¹⁷⁷Lu@NaYF₄@LI (NPs-¹⁷⁷Lu) and ¹⁷⁷LuCl₃ (¹⁷⁷Lu) were separately incubated with U87MG cells at 37 °C for different periods of time (6 h, 12 h, and 24 h), and the binding of the two drugs to U87MG cells was investigated. As shown in FIG. 15, NaGdF₄@NaYbF₄:Er/Ce/¹⁷⁷Lu@NaYF₄@LI could rapidly bind to U87MG cells. After 6 h of incubation, the binding rates of NaGdF₄@NaYbF₄:Er/Ce/¹⁷⁷Lu@NaYF₄@LI and ¹⁷⁷LuCl₃ to U87MG cells were 28.5±3.5% and 2.0±0.1%, respectively. As the incubation time increased, the binding rates of NaGdF₄@NaYbF₄:Er/Ce/¹⁷⁷Lu@NaYF₄@LI to U87MG cells tended to increase slightly. After 24 h of incubation, the binding rates of NaGdF₄@NaYbF₄:Er/Ce/¹⁷⁷Lu@ NaYF₄@ LI and ¹⁷⁷LuCl₃ to U87MG cells were 39.2±2.5% and 2.4±0.8%, respectively. Comparing the differences in binding of NaGdF₄@NaYbF₄:Er/Ce/¹⁷⁷Lu@NaYF₄@LI and ¹⁷⁷LuCl₃ to U87MG cells, it is further shown that NaGdF₄@NaYbF₄:Er/Ce/¹⁷⁷Lu@NaYF₄@LI has a good targeting property on U87MG cells.

### (3) In vitro cell killing assay of NaGdF₄@NaYbF₄:Er/Ce@NaYF₄:⁹⁰Y@LI

U87MG cells were seeded in a 96-well plate at a density of 3 × 10⁴ cells/well. After the cells completely adhered to the wall, different doses (0.2 mCi, 0.39 mCi, 0.79 mCi, 1.58 mCi, 3.15 mCi, and 6.3 mCi) of NaGdF₄@NaYbF₄:Er/Ce@NaYF₄:⁹⁰Y@LI (NPs-⁹⁰Y), ⁹⁰YCl₃, or NaGdF₄@NaYbF₄:Er/Ce@NaYF₄ (NPs) were added. Three replicate wells were set for each group. After incubation at 37 °C for 24 h, the old medium was discarded. The medium was replaced with a fresh one, and then 10 µL of a CCK-8 working solution was added to each well. The plate was incubated in an incubator at 37 °C for 2 h. Then the absorbance value at 450 nm was detected using a microplate reader, and the cell proliferation activity of each group was calculated. Cell viability (%) = [(OD value of administration group - OD value of blank group)/(OD value of control group - OD value of blank group)] × 100%.

Under the condition of the same dose, the NPs without the nuclide (400 µg/mL) and the pure nuclide had almost no killing effect on U87MG (6.3 µCi) cells, and the cell viabilities of both were 90% or more (FIG. 16), in sharp contrast to the NPs-⁹⁰Y (200 µg/mL, 6.3 µCi) in which the viability of U87MG cells was only 65% after 24 h of treatment. This fully demonstrates that our rare earth nano-fluorescence-nuclear medicine tumor imaging-therapy integrated formulation has a strong killing ability on U87MG cells at a low irradiation dose.

### (4) In vitro cell killing assay of NaGdF₄@NaYbF₄:Er/Ce/¹⁷⁷Lu@NaYF₄@LI

U87MG cells were seeded in a 96-well plate at a density of 3 × 10⁴ cells/well. After the cells completely adhered to the wall, different doses (0.4 mCi, 0.8 mCi, 1.6 mCi, 3.2 mCi, and 6.4 mCi) of NaGdF₄@NaYbF₄:Er /Ce/¹⁷⁷Lu @NaYF₄@LI, ¹⁷⁷LuCl₃, or NaGdF₄@NaYbF₄:Er/Ce @NaYF₄ (NPs) were added. Three replicate wells were set for each group. After incubation at 37 °C for 24 h, the old medium was discarded. The medium was replaced with a fresh one, and then 10 µL of a CCK-8 working solution was added to each well. The plate was incubated in an incubator at 37 °C for 2 h. Then the absorbance value at 450 nm was detected using a microplate reader, and the cell proliferation activity of each group was calculated. Cell viability (%) = [(OD value of administration group - OD value of blank group)/(OD value of control group - OD value of blank group)] × 100%.

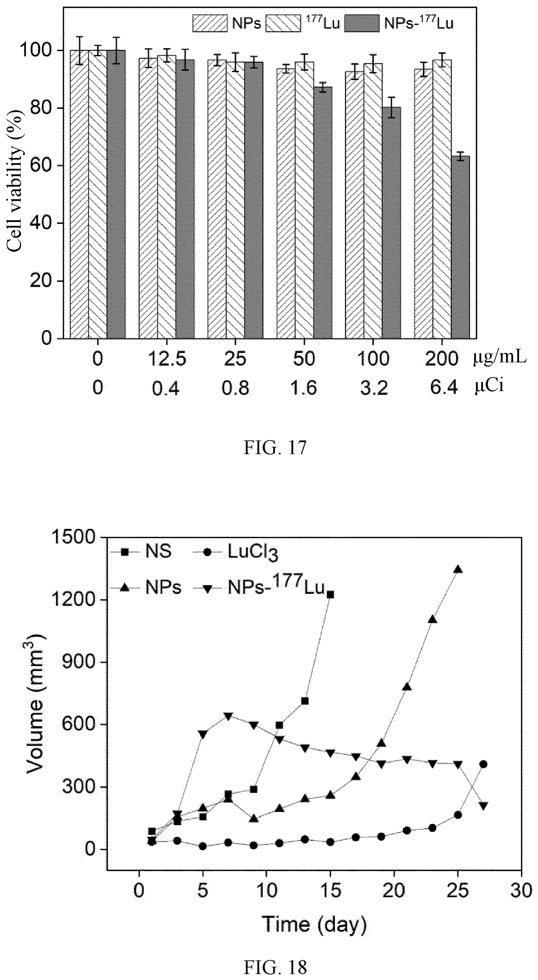

Under the condition of the same dose, the NPs without the nuclide (200 µg/mL) and the pure nuclide had almost no killing effect on U87MG (6.4 µCi) cells, and the cell viabilities of both were 95% or more (FIG. 17), in sharp contrast to the NPs-¹⁷⁷Lu (200 µg/mL, 6.4 µCi) in which the viability of U87MG cells was only 64% after 24 h of treatment. This fully demonstrates that our rare earth nano-fluorescence-nuclear medicine tumor imaging-therapy integrated formulation has a strong killing ability on U87MG cells at a low irradiation dose.

### (5) Evaluation of in vivo anti-tumor effect of NaGdF₄@NaYbF₄:Er/Ce/¹⁷⁷Lu@NaYF₄@LI

Nude mice bearing U87MG subcutaneous tumors were randomized into 4 groups of 5 mice each and treated with NS (0.9% NaCl), ¹⁷⁷LuCl₃ (100 µCi), NPs (150 mg/kg), and NPs-¹⁷⁷Lu (100 µCi, 150 mg/kg), respectively. The route of administration was intratumoral injection. After the treatment, the tumor size and the body weight of the nude mice were measured once every 2 days. During the treatment, the mice were sacrificed when the tumor volume exceeded 1 cm³ or the body weight loss exceeded 20%. At the end of the 21-day observation period, the nude mice were euthanized. Important tissues and organs such as the heart, liver, spleen, lung, kidney, and tumor were carefully isolated, completely immersed in a 4% paraformaldehyde tissue fixative solution, and stored in a refrigerator at 4 °C. After being completely free of radioactivity, the fixed tumor tissues were subjected to HE and TUNEL assays.

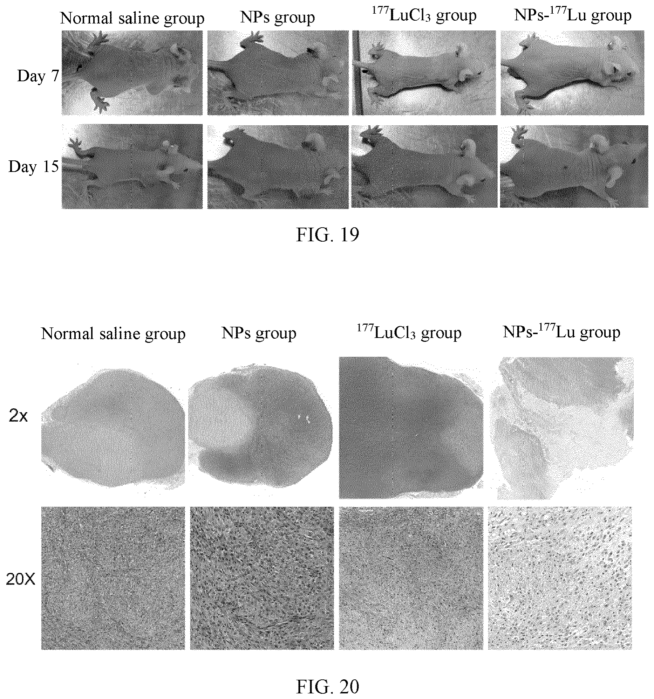

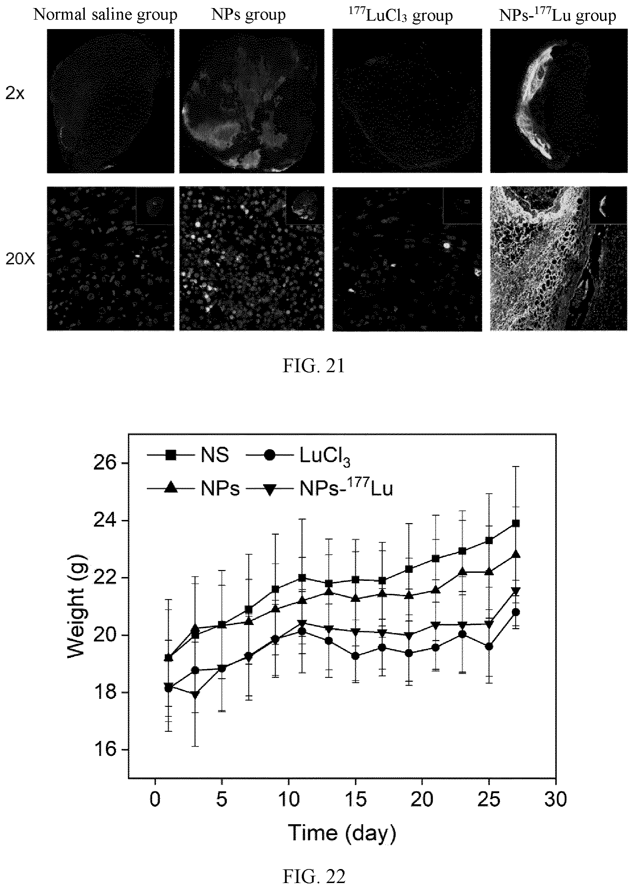

According to the tumor growth curves (FIG. 18) and the tumor treatment effect graph of each group (FIG. 19) during the treatment, the average tumor volumes of the NS group and the NPs group continuously increased and reached the sacrifice criteria on day 14 and day 21, respectively. The tumor volume of the ¹⁷⁷LuCl₃ group increased slowly in the early stage and increased significantly in the later stage of the treatment, and it is predictable that the tumor will definitely exceed 1,000 mm³. The average tumor volume of the NPs-¹⁷⁷Lu group increased significantly in the early stage, which was caused by the swelling and inflammation of the tumor tissues after the injection of the drug; the average tumor volume began to slowly decrease after day 7, accompanied by the phenomenon of scab falling off; by the end of the treatment period, the tumor almost disappeared, and the volume shown in the figure was a scar after the scab of the tumor. This can also be proved from the HE (FIG. 20) results of the tumor. The tumor cell density of the normal saline group was large, and the nuclear morphology was complete; in the NPs group, there were vacuoles formed by apoptosis in a small part of the area, and the tumor cell density was also slightly low, which may be the initial administration site, causing the death of some tumor cells; in the ¹⁷⁷LuCl₃ group, the tumor cell density was slightly low, there were also vacuoles formed by apoptosis in a small part of the area, and the tumor cell morphology in the remaining areas was intact, indicating that the pure nuclide therapy is likely to result in recurrence; in the NPs-¹⁷⁷Lu group, substantially no intact tumor cells could be found, and there were a large area of vacuoles formed by tumor cell apoptosis at the tumor site. The tumor tissue TUNEL assay (FIG. 21) was used to evaluate the apoptosis of tumor cells. The normal saline group showed only sporadic apoptosis, which was caused by normal apoptosis of the cells. Surprisingly, the NPs group showed relatively high apoptosis expression in tumors, and the apoptosis expression exhibited an uneven and block-like distribution pattern, which may be due to different degrees of embolization formed by the nanoparticles in the tumor tissues, resulting in necrosis in some areas. The ¹⁷⁷LuCl₃ group showed only a small amount of apoptosis expression, which may be because the pure nuclide had lost its efficacy when the treatment period was too long. In sharp contrast, the NPs-¹⁷⁷Lu group showed a large amount of apoptosis expression, and the part not shown in green was a normal tissue that was cut off because the tumor was too small.

Therefore, the experimental results described above can demonstrate that the rare earth nano-fluorescence-nuclear medicine tumor imaging-therapy integrated formulation prepared by the present disclosure has good *in vivo* anti-tumor ability.

### Experimental Example 6

### Biological safety of NaGdF₄@NaYbF₄:Er/Ce/¹⁷⁷Lu@NaYF₄@LI rare earth nano-fluorescence-nuclear medicine tumor imaging-therapy integrated formulation

Changes in the body weight of the nude mice were recorded while the tumor volume was recorded to evaluate the potential toxicity of NaGdF₄@NaYbF₄:Er/Ce/¹⁷⁷Lu@NaYF₄@LI. In addition, after the treatment period was over, the heart blood was collected and centrifuged to isolate the plasma. The blood of the nude mice was centrifuged at 4,000 rpm for 10 min, and the supernatant was carefully pipetted and centrifuged at 12,000 rpm at 4 °C for another 15 min. The upper-layer serum was taken to measure blood glucose and liver and kidney functions. Important tissues and organs such as the heart, liver, spleen, lung, and kidney were carefully isolated, fixed with 4% paraformaldehyde, embedded in paraffin, sectioned, and then stained with HE for histopathological examination.

FIG. 22 shows the trend of changes in the body weight of the nude mice in each group during the treatment. The body weight is an important indicator reflecting the health condition of the nude mice. As can be seen from the figure, the body weights of the nude mice in all groups showed an increasing trend during the treatment, indicating that our drug does not cause significant damage to the body of the nude mice.

Table 1 shows the blood routine examination results of the nude mice in all groups after the treatment of the nude mice with the NaGdF₄@NaYbF₄:Er/Ce/¹⁷⁷Lu@NaYF₄@LI tumor imaging-therapy integrated formulation in Example 1, NaGdF₄@NaYbF₄:Er/Ce@NaYF₄@LI in Comparative Example 1, ¹⁷⁷LuCl₃, and normal saline.

Table 2 shows the blood biochemical results of the nude mice in all groups after the treatment of the nude mice with the NaGdF₄@NaYbF₄:Er/Ce/¹⁷⁷Lu@NaYF₄@LI tumor imaging-therapy integrated formulation in Example 1, NaGdF₄@NaYbF₄:Er/Ce@NaYF₄@LI in Comparative Example 1, ¹⁷⁷LuCl₃, and normal saline.

**Table 1.**

| **Item name** | Unit | **Normal** saline group | **NPs** group | **¹⁷⁷ LuCl₃** group | NPs-¹⁷⁷Lu group |
|---|---|---|---|---|---|
| Number of leukocytes | 10^9/L | 191.77 | 191.77 | 203.65 | 195.73 |
| Number of neutrophils | 10^9/L | 178.94 | 178.94 | 194.89 | 185 |
| Number of lymphocytes | 10^9/L | 9.93 | 9.93 | 7.71 | 9.19 |
| Number of monocytes | 10^9/L | 2.89 | 2.89 | 1.04 | 2.28 |
| Number of red blood cells | 10^12/L | 7.03 | 7.03 | 6.27 | 6.78 |
| Hemoglobin concentration | g/L | 119.25 | 119.25 | 103.33 | 113.94 |
| Hematocrit | % | 34.98 | 34.98 | 30.55 | 33.50 |
| Mean corpuscular volume | fL | 49.75 | 49.75 | 49.33 | 49.61 |
| Mean corpuscular hemoglobin | pg | 17.03 | 17.03 | 16.78 | 16.94 |
| Mean corpuscular hemoglobin concentration | g/L | 342.25 | 342.25 | 339.50 | 341.33 |
| Red cell distribution width-coefficient of variation | % | 14.60 | 14.60 | 16.10 | 15.10 |
| Number of platelets | 10^9/L | 657.25 | 657.25 | 860.67 | 725.06 |
| Mean platelet volume | fL | 7.00 | 7.00 | 6.28 | 6.76 |

As can be seen from Table 1, the blood routine examination results show that all nude mice had different degrees of inflammation, but no statistically significant difference was shown between the different groups.

**Table 2.**

| **Item** | Unit | **Normal** saline group | **NPs** group | **¹⁷⁷ LuCl₃** group | NPs-¹⁷⁷Lu group |
|---|---|---|---|---|---|
| Creatinine | µmol/L | 17.55 | 14.93 | 17.97 | 13.57 |
| Urea | mmol/L | 7.58 | 7.73 | 6.72 | 7.49 |
| Blood urea nitrogen | mmol/L | 3.42 | 3.48 | 3.04 | 3.45 |
| Aspartate aminotransferase | U/L | 206.38 | 223.25 | 269.90 | 178.93 |
| Alanine aminotransferase | U/L | 24.10 | 27.38 | 72.70 | 30.83 |
| Blood glucose | mmol/L | 10.12 | 9.99 | 10.12 | 8.13 |
| Total cholesterol | mmol/L | 1.35 | 1.59 | 1.61 | 1.68 |
| Lactate dehydrogenase | mmol/L | 728.73 | 1138.83 | 1380.90 | 493.43 |

The blood biochemical results in Table 2 show that the blood glucose and blood lipid indexes of all groups were normal, and the renal function index of the ¹⁷⁷LuCl₃ group and the liver function index of the NPs-¹⁷⁷Lu group were abnormal.

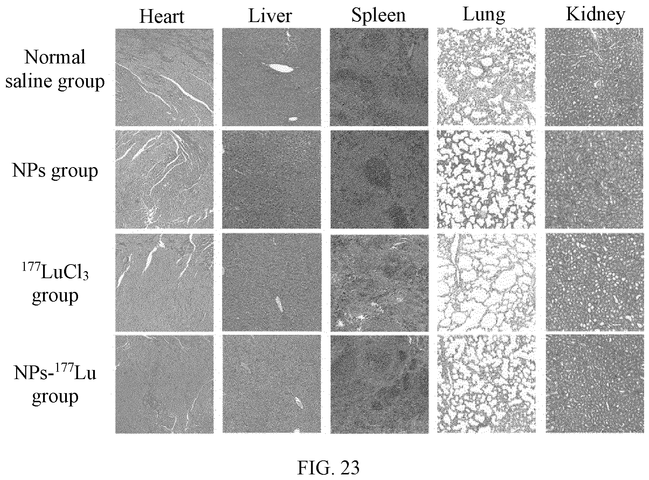

FIG. 23 shows pathological sections of the tissues of representative nude mice selected from each group. The main organs of the nude mice in each group were observed by sectioning, and it was found that the glomerular cells in the ¹⁷⁷LuCl₃ group were irregular in shape, the hepatocytes in the NPs-¹⁷⁷Lu group did have mild inflammation and enlargement, and there was no significant difference in the other organs from the negative control group, but the side effects at this degree were acceptable.

It can be seen that the rare earth nano-fluorescence-nuclear medicine tumor imaging-therapy integrated formulation prepared by the present disclosure has good biological safety in addition to good imaging and therapeutic effects.

The exemplary embodiments of the present disclosure have been described above. However, the protection scope of the present application is not limited to the embodiments described above. Any modification, equivalent, improvement, and the like made by those skilled in the art without departing from the spirit and principle of the present disclosure shall fall within the protection scope of the present application.

## Claims

1. A rare earth nano-fluorescence-nuclear medicine tumor imaging-therapy integrated formulation, comprising a rare earth inorganic nanocrystal with a microscopic core-shell-shell structure, a rare earth medical nuclide A, and a functional tumor-targeting biomolecule loaded on the surface of the rare earth inorganic nanocrystal, wherein the rare earth medical nuclide A is at least distributed inside the microscopic core-shell-shell nanostructure and/or coupled to the functional tumor-targeting biomolecule;
the imaging-therapy integrated formulation has a general structural formula of A-MLnF₄@MLnF₄:Em@MLnF₄@ LI;
wherein the rare earth medical nuclide A is selected from one or more of ¹⁷⁷Lu, ¹⁶⁹Er, ¹⁵³Sm, ⁹⁰Y, ⁸⁶Y, ⁴⁴Sc, and ⁴⁷Sc;
M is selected from one or more of metal elements Li, Na, K, Rb, Cs, Mg, Ca, Sr, and Ba;
Ln is a stable rare earth element selected from one or more of La, Ce, Pr, Nd, Po, Sm, Eu, Gd, Tb, Dy, Ho, Er, Tm, Yb, Lu, Y, and Sc, preferably one or more of Yb, Er, Tm, Ho, Gd, Y, Ce, and Lu, exemplarily one or more of Gd, Y, Yb, Er, and Ce;
Em is a rare earth luminescent ion selected from one or more of elemental ions Pr, Nd, Sm, Eu, Tb, Dy, Ho, Er, Tm, and Yb; and
LI is the functional tumor-targeting biomolecule.

2. The rare earth nano-fluorescence-nuclear medicine tumor imaging-therapy integrated formulation according to claim 1, wherein the rare earth medical nuclide A is ¹⁷⁷Lu or ⁹⁰Y.

3. The rare earth nano-fluorescence-nuclear medicine tumor imaging-therapy integrated formulation according to claim 1, wherein the rare earth inorganic nanocrystal with the microscopic core-shell-shell structure comprises a rare earth ion inner core, a rare earth ion intermediate shell layer, and a rare earth ion inert protective layer, and the rare earth medical nuclide A is at least distributed on at least a portion of the rare earth ion inner core, the rare earth ion intermediate shell layer, and the rare earth ion inert protective layer.

4. The rare earth nano-fluorescence-nuclear medicine tumor imaging-therapy integrated formulation according to claim 1, wherein the **LI** is from a biomolecule with tumor-specific recognition capability coordinated with a water-soluble ligand without tumor-specific recognition capability;
preferably, the water-soluble ligand without tumor-specific recognition capability and the biomolecule with tumor-specific recognition capability are in a mass ratio of (0-100):1, preferably (0-10):1, for example, 0:1, 1:1, 2:1, 3:1, 4:1, 5:1, 6:1, 7:1, 8:1, 9:1, 10:1, or any range between any of the values described above;
preferably, the biomolecule with tumor-specific recognition capability is selected from one or more of an organic small molecule, an antibody, a polypeptide, and a single- or multi-target small molecule inhibitor, more preferably one or more of a monoclonal antibody, a polyclonal antibody, a polysaccharide, a nucleic acid, folic acid, aspirin, and vitamin C;
preferably, the water-soluble ligand without tumor-specific recognition capability is selected from an amphiphilic copolymer, further preferably a diblock copolymer and/or a triblock copolymer;
for example, a hydrophilic group in the amphiphilic copolymer is one or more of polyethylene glycol (PEG), monomethoxy poly(ethylene glycol) (mPEG), polyvinylpyrrolidone (PVP), polyoxyethylene (PEO), polyvinyl alcohol (PVA), polyacrylic acid (PAA), chitosan and derivatives thereof, cell membrane-mimetic phosphorylcholine (PC), and water-soluble cyclodextrin derivatives;
preferably, the polyethylene glycol preferably has a number-average molecular weight of 300-50,000, more preferably 500-10,000, such as 500, 1,000, 2,000, 3,000, 4,000, 5,000, 5,500, 6,000, 6,500, 7,000, 7,500, 8,000, 8,500, 9,000, 9,500, 10,000, 20,000, 25,000, 30,000, 40,000, 50,000, or any range between any two of the values;
preferably, a hydrophobic group in the amphiphilic copolymer is preferably one or more of polylactic acid, poly-L-lactic acid, poly-D,L-lactic acid, polylactide-glycolide, poly-ε-caprolactone, poly(benzyl aspartate), poly(benzyl glutamate), polystyrene, poly(isopropylacrylamide), polylysine, polyaspartic acid, polyhistidine, phospholipid, phosphatidylethanolamine, distearoyl phosphatidyl ethanolamine, cholesterol, and a hydrophobic cyclodextrin derivative, for example, one or more of polyethylene glycol-polylactic acid, polyethylene glycol-poly-D-lactic acid, polyethylene glycol-poly-L-lactic acid, polyethylene glycol-poly-D,L-lactic acid, polyethylene glycol-polylactide-glycolide, distearoyl phosphatidyl ethanolamine-polyethylene glycol and derivatives thereof, dipalmitoyl phosphatidyl ethanolamine-polyethylene glycol and derivatives thereof, dilauroyl phosphatidyl ethanolamine-polyethylene glycol and derivatives thereof, dimyristoyl phosphatidyl ethanolamine-polyethylene glycol and derivatives thereof, and dioleoyl phosphatidyl ethanolamine-polyethylene glycol and derivatives thereof.

5. The rare earth nano-fluorescence-nuclear medicine tumor imaging-therapy integrated formulation according to claim 1, wherein the imaging-therapy integrated formulation is
NaGdF₄@NaYbF₄:Er/Ce/¹⁷⁷Lu@NaYF₄ nanomaterial, NaGdF₄@NaYbF₄:Er/Ce/¹⁶⁹Er@NaYF₄ nanomaterial, NaGdF₄@NaYbF₄:Er/Ce/⁹⁰Y@NaYF₄ nanomaterial, NaGdF₄@NaYbF₄:Er/Ce/¹⁵³Sm@NaYF₄ nanomaterial, NaGdF₄@NaYbF₄:Er/Ce/⁸⁶Y@NaYF₄ nanomaterial, NaGdF₄@NaYbF₄:Er/Ce/⁴⁴Sc@NaYF₄ nanomaterial, NaGdF₄@NaYbF₄:Er/Ce/⁴⁷Sc@NaYF₄ nanomaterial, NaYF₄@NaYbF₄:Er/Ce/¹⁷⁷Lu@NaYF₄ nanomaterial, NaYF₄@NaYbF₄:Er/Ce/¹⁶⁹Er@NaYF₄ nanomaterial, NaYF₄@NaYbF₄:Er/Ce/⁹⁰Y@NaYF₄ nanomaterial, NaYF₄@NaYbF₄:Er/Ce/¹⁵³Sm@NaYF₄ nanomaterial, NaYF₄@NaYbF₄: Er/Ce/^{g6}Y@NaYF₄ nanomaterial, NaYF₄@NaYbF₄:Er/Ce/⁴⁴Sc@NaYF₄ nanomaterial, NaYF₄@NaYbF₄:Er/Ce/⁴⁷Sc@NaYF₄ nanomaterial, NaGdF₄:¹⁷⁷Lu@NaYbF₄:Er/Ce@NaYF₄ nanomaterial, NaGdF₄:¹⁶⁹Er@NaYbF₄:Er/Ce@NaYF₄ nanomaterial, NaGdF₄:⁹⁰Y@NaYbF₄:Er/Ce@NaYF₄ nanomaterial, NaGdF₄:¹⁵³Sm@NaYbF₄:Er/Ce/@NaYF₄ nanomaterial, NaGdF₄:⁸⁶Y@NaYbF₄:Er/Ce@NaYF₄ nanomaterial, NaGdF ₄:⁴⁴Sc@NaYbF₄:Er/Ce@NaYF₄ nanomaterial, NaGdF₄:⁴⁷Sc@NaYbF₄:Er/Ce@NaYF₄ nanomaterial, NaGdF₄@NaYbF₄:Er/Ce@NaYF₄:¹⁷⁷Lu nanomaterial, NaGdF₄@NaYbF₄:Er/Ce@NaYF₄:¹⁶⁹Er nanomaterial, NaGdF₄@NaYbF₄:Er/Ce@NaYF₄:⁹⁰Y nanomaterial, NaGdF₄@NaYbF₄:Er/Ce/@NaYF₄:¹⁵³Sm nanomaterial, NaGdF₄@NaYbF₄:Er/Ce@NaYF₄:⁸⁶Y nanomaterial, NaGdF₄@NaYbF₄:Er/Ce@NaYF₄:⁴⁴Sc nanomaterial, NaGdF₄@NaYbF₄:Er/Ce@NaYF₄:⁴⁷Sc nanomaterial, NaYF₄@NaYF₄:Yb/Er/Ce/¹⁷⁷Lu@NaYF₄ nanomaterial, NaYF₄@NaYF₄:Yb/Er/Ce/¹⁶⁹Er@NaYF₄ nanomaterial, NaYF₄@NaYF₄:Yb/Er/Ce/⁹⁰Y@NaYF₄ nanomaterial, NaYF₄@NaYF₄:Yb/Er/Ce/¹⁵³Sm@NaYF₄ nanomaterial, NaYF₄@NaYF₄:Yb/Er/Ce/⁸⁶Y@NaYF₄ nanomaterial, NaYF₄@NaYF₄:Yb/Er/Ce/⁴⁴Sc@NaYF₄ nanomaterial, NaYF₄@NaYF₄:Yb/Er/Ce/⁴⁷Sc@NaYF₄ nanomaterial, KGdF₄@KYbF₄:Er/Ce/¹⁷⁷Lu@KYF₄ nanomaterial, or LiGdF₄@LiYbF₄:Er/Ce/¹⁷⁷Lu@LiYF₄ nanomaterial.

6. The rare earth nano-fluorescence-nuclear medicine tumor imaging-therapy integrated formulation according to any one of claims 1 to 5, wherein the imaging-therapy integrated formulation has a size of 10-200 nm, preferably 20-100 nm, for example, 10 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, or any range between any two of the points described above;
preferably, the functional tumor-targeting biomolecule has a thickness of 1-100 nm, preferably 5-50 nm.

7. A preparation method for the rare earth nano-fluorescence-nuclear medicine tumor imaging-therapy integrated formulation according to any one of claims 1 to 6, comprising:
S1, obtaining a rare earth ion inner core by a high-temperature thermal decomposition method;
S2, enabling a rare earth ion intermediate shell layer and a rare earth ion inert protective layer to grow step by step on the surface of the rare earth ion inner core by adopting a layer-by-layer epitaxial growth method; and
S3, labeling a rare earth medical nuclide A to at least a portion of the rare earth ion inner core, the rare earth ion intermediate shell layer, and the rare earth ion inert protective layer as required, and then loading a functional tumor-targeting biomolecule on the surface of the rare earth ion inert protective layer; or
loading a functional tumor-targeting biomolecule on the surface of the rare earth ion inert protective layer, and then labeling a rare earth medical nuclide A on the functional tumor-targeting biomolecule to obtain the tumor imaging-therapy integrated formulation.

8. The preparation method according to claim 7, comprising the following steps:
(a) adding a stable rare earth element, an alkali metal source, a long-alkyl-chain organic acid, and a long-alkyl-chain olefin into a reaction vessel, mixing until dissolved, and then performing a high-temperature thermal decomposition reaction to obtain a nanocrystal rare earth ion inner core;
(b) adding the nanocrystal rare earth ion inner core, the stable rare earth element, the alkali metal source, the long-alkyl-chain organic acid, and the long-alkyl-chain olefin into a reaction vessel, mixing until dissolved, and then performing a high-temperature thermal decomposition reaction to obtain a nanocrystal with a core-shell structure;
(c) adding the nanocrystal with the core-shell structure, the stable rare earth element, the alkali metal source, the long-alkyl-chain organic acid, and the long-alkyl-chain olefin into a reaction vessel, mixing until dissolved, and then performing a high-temperature thermal decomposition reaction to obtain a rare earth nano-fluorescence-nuclear medicine tumor imaging-therapy integrated formulation precursor with a core-shell-shell structure; and
(d) modifying the surface of the imaging-therapy integrated formulation precursor with the functional tumor-targeting biomolecule,
wherein preferably, the rare earth medical nuclide A is loaded in at least one process of step (a), step (b), step (c), and step (d);
preferably, the rare earth luminescent ion Em is added in at least one process of step (a), step (b), and step (c);
further preferably, the rare earth luminescent ion Em is added in step (b);
preferably, the temperature is raised to 280-340 °C, and the reaction is performed for 0.5-2 h in step (a), step (b), and step (c);
preferably, the step of modifying with the functional tumor-targeting biomolecule in step (d) comprises:
dispersing the imaging-therapy integrated formulation precursor in an organic solvent, adding distearoyl phosphatidyl ethanolamine-polyethylene glycol and a polypeptide derivative thereof, stirring to convert the nanocrystal from oil-soluble to water-soluble, completely volatilizing the solvent naturally, and then adding a proper amount of water for ultrasonic mixing to obtain the rare earth nano-fluorescence-nuclear medicine tumor imaging-therapy integrated formulation.

9. The preparation method according to claim 7, wherein the stable rare earth element is selected from one or more of an acetate, a chloride salt, and a nitrate of a rare earth ion stable isotope Ln, preferably a trifluoroacetate and a rare earth chloride salt;
the stable rare earth element is selected from trifluoroacetates and/or chloride salts of ytterbium, erbium, gadolinium, thulium, holmium, samarium, neodymium, yttrium, and lutetium, exemplarily ytterbium trifluoroacetate, yttrium trifluoroacetate, erbium trifluoroacetate, cerium trifluoroacetate, and gadolinium trifluoroacetate;
preferably, the alkali metal source is selected from one or more of salts, hydroxides, and bifluorides containing an M element; M is selected from one or more of metal elements Li, Na, K, Rb, Cs, Mg, Ca, Sr, and Ba;
further preferably, the alkali metal source is an oleate, an acetate, or a fluoride containing the M element, such as a trifluoroacetate, exemplarily a trifluoroacetate or a bifluoride;
preferably, the long-alkyl-chain organic acid is selected from one or more of octanoic acid, lauric acid, and oleic acid, exemplarily oleic acid;
preferably, the long-alkyl-chain olefin is selected from one or more of 1-decene, **1-**dodecene, and 1-octadecene, exemplarily 1-octadecene;
preferably, the rare earth medical nuclide A source is selected from an oxalate of a radioisotope and/or a chloride salt of a radioisotope, exemplarily ¹⁷⁷LuCl₃ and ⁹⁰YCl₃; preferably, the stable rare earth element, the long-alkyl-chain organic acid, and the long-alkyl-chain olefin are in a molar volume ratio of (0.01-0.4) mmol:(1-20) mL:(1-20) mL, further preferably (0.01-0.2) mmol:(2-10) mL:(2-10) mL;
preferably, the rare earth medical nuclide A and the stable rare earth element are in a radioactivity-to-molar ratio of (0.01-100) mCi:(0.01-0.4) mmol, further preferably (1-50) mCi:(0.01-0.1) mmol;
preferably, the organic solvent is selected from one or more of cyclohexane, dichloromethane, trichloromethane, tetrahydrofuran, and N,N-dimethylformamide;
preferably, the nanocrystal, distearoyl phosphatidyl ethanolamine-polyethylene glycol and the polypeptide derivative of distearoyl phosphatidyl ethanolamine-polyethylene glycol, cyclohexane, and trichloromethane are in a molar volume ratio of (0.01-0.2) mmol:(0.2-0.8) mmol:(1-10) mL:(2.5-10) mL, preferably (0.05-0.1) mmol:(0.3-0.5) mmol:(1-3) mL:(8-10) mL.

10. Use of the rare earth nano-fluorescence-nuclear medicine tumor imaging-therapy integrated formulation according to any one of claims 1 to 6 as a rare earth fluorescent medical imaging agent, particularly as a rare earth fluorescent medical imaging agent for solid tumor lesions or small and micro metastatic tumor lesions,
wherein preferably, the rare earth nano-fluorescence-nuclear medicine tumor imaging-therapy integrated formulation is used as a rare earth nano-fluorescent tumor medical imaging agent, a nuclear medicine tumor imaging agent, and a tumor radiotherapeutic agent, and as the integration of tumor diagnosis and treatment under the combined guidance of rare earth nano-fluorescence-nuclear medicine multimodal medical imaging.
